# EUROPEAN PATENT APPLICATION

(11) **EP 4 541 983 A1**
(43) Date of publication of application: **23.04.2025**
(21) Application number: 24207037.3
(22) Date of filing: 16.10.2024
(51) Int. Cl.: E04C 2/04, A01G 18/00, E04C 2/16, E04C 2/38, E04C 2/40, C12N 1/14

(54) **METHOD OF MANUFACTURING A PREFAB CONSTRUCTION ELEMENT**

(30) Priority: 16.10.2023 EP 23203887; 08.03.2024 EP 24162356
(71) Applicant: Comfortdak B.V., 3707 TB Zeist (NL)
(72) Inventor: Branderhorst, Rudolf Hendrik, 5037 BA Tilburg (NL); Borra, Hans Antonius, 3707 TB Zeist (NL)
(74) Representative: De Vries & Metman

(57) **Abstract**

The invention relates to a method of manufacturing a prefab construction element, preferably a load-bearing element, such as a roofing, flooring or wall panel (14), comprising the steps of providing one or more beams, preferably load-bearing beams (1), providing a plurality of blocks (11) of mycelium composite, and assembling the prefab construction element (14) from the one or more load-bearing beams (1) and a plurality of the blocks (11).

## Description

The invention relates to a method of manufacturing a prefabricated (prefab) construction element, preferably a load-bearing construction element, such as a roofing, flooring or wall panel. The invention further relates to a kit for manufacturing a prefab construction element and to an assembled prefab construction element.

WO 2008/073489 relates to a composite material that is comprised of a substrate of discrete particles and a network of interconnected mycelia cells bonding the discrete particles together. The composite material is made by inoculating a substrate of discrete particles and a nutrient material with a preselected fungus. The fungus digests the nutrient material over a period of time sufficient to grow hyphae and to allow the hyphae to form a network of interconnected mycelia cells through and around the discrete particles thereby bonding the discrete particles together to form a self-supporting composite material.

In the example shown in Figure 7 of WO 2008/073489, stiff exterior faces are added to a rectangular panel, thus a "panelized system composed of a mycelia bonded core and exterior facing system can be created. This panelized system has superior strength characteristics due to the addition of stiff exterior faces."

"In another embodiment, samples have also been produced where the exterior faces are placed *in vitro* during the incubator process. The growth of the filamentous fungi directly bonds the exterior faces to the mycelia bonded composite core producing a panelized system that can be used immediately after drying."

WO 2018/014004 relates to forming fungal materials and fungal objects from those fungal materials, the method comprising the steps of growing a first fungal tissue in contact with a nutritive vehicle; supplying a porous material in contact with said first fungal tissue; directing growth of said fungal tissue through said porous material such that a portion of said fungal tissue comprises a first fungal material having first fungal hyphae; optionally incorporating composite material; directing a change in the composition or growth pattern of at least some of said first fungal hyphae; separating at least a portion of the first fungal material from said nutritive vehicle; obtaining a second fungal material having second fungal hyphae; and forming a fungal object by encouraging fused growth between said first fungal material and said second fungal material and optionally incorporating composite material.

It is an object of the present invention to provide an improved method of manufacturing a prefabricated construction element.

To this end, the method according to the present invention comprises the steps of providing one or more beams, preferably load-bearing beams, such as rafters, providing a plurality of blocks of mycelium composite, and assembling the prefab construction element from the one or more load-bearing beams and a plurality of the blocks.

An embodiment comprises the steps of placing two or more beams alongside each other, preferably parallel, and placing a plurality of the blocks between these beams, preferably with the blocks abutting each other and/or filling the space between the beams, preferably entirely.

An embodiment comprises the step of interconnecting the beams, e.g. by means of a base plate or frame and preferably before placing the blocks. In an embodiment, the beams are adhered to the base plate or frame, e.g. by means of an adhesive, or mechanically fastened to the base plate or frame, e.g. by means of screws, nails or staples.

To provide particularly rigid elements, an embodiment comprises the step of placing a cover, e.g. a top plate, over the placed beams and blocks and fastening the cover to the beams and/or to the blocks.

In an embodiment, the method comprises the steps of providing an opening in the construction element and placing one or more mounting elements, e.g. solid blocks made from wood, wood fibers, or a synthetic material, between the beams and/or in the ends of the beams.

In an embodiment, the mounting elements have a cross-section that corresponds to the cross-section of the blocks of mycelium composite or to the cross-section of the elongated enclosures of the beams and preferably fit snugly between the beams or in the elongated enclosures of the beams and/or, after placing, sit flush with the edge of the ends of the beams and thus flush with the opening.

The mounting elements enable a very efficient way of providing an opening and frame in the construction element, e.g. at the factory but or at a building site, if required.

By removing mycelium composite from the mycelium block(s) and/or from the elongated enclosure(s) - or by providing less mycelium in the first place - over a height that corresponds to the height of the mounting elements, the latter, once placed in the resulting cavities, sit flush with the edge of the ends of the beams and thus flush with the edge of the opening for the window. The mounting elements are fixed in place e.g. by means of nails driven through an outer wall of the panel or beams or by means of an adhesive. Once the mounting elements have been placed and fixed to the beams, a frame can be placed in the opening and fixed, e.g. by screws, to the mounting elements.

The invention also relates to a kit for manufacturing a prefab construction element, preferably a load-bearing element, such as a roofing, flooring or wall panel, the kit comprising one or more beams, preferably load-bearing beams, a plurality of blocks of mycelium composite, and preferably a base plate or frame for interconnecting the beams and/or mounting elements to be fitted between the beams and/or in the ends of the beams.

The present invention enables or at least facilitates large scale use of mycelium in prefab load-bearing and/or insulating construction elements, in that, on the one hand, the blocks can be produced in a relatively efficient, reproducible, homogenous and/or relatively fast manner and, on the other hand, the load-bearing prefab elements can be made in large sizes and preferably of at least 50 volume-percent of mycelium, preferably at least 70 volume-percent of mycelium. Also, the present invention provides a modern and optionally 100% organic construction element and kit that in principle fit substantially seamlessly in current prefab logistics.

The beams can be made e.g. of wood. In an embodiment, one or more of the load-bearing beams comprises an elongated enclosure which enclosure contains a mixture of at least one fungus and a substrate, wherein a network of hyphae has formed through the mixture and into the walls of the enclosure to form a mycelium composite.

Such beams can be obtained for instance by introducing or preparing a mixture of a fungus and a substrate in the enclosure and allowing the fungus to grow to form a network of hyphae through the mixture and into the walls of the enclosure to form a mycelium composite, and drying the composite, preferably while it remains in the enclosure.

The elongated enclosure can be a single, continuous enclosure or comprise a plurality of enclosures, such as compartments.

In an embodiment, one or more beams are at least 2 meter (m) long, preferably at least 4 m long, and/or less than 0,4 m wide, preferably less than 0,3 m wide, and/or less than 0,5 m high, preferably less than 0,35 m high.

In an embodiment, the blocks are at least 0,3 m long, preferably at least 0,4 m long, and/or at least 0,3 m wide, preferably less than 0,4 m wide, and/or less than 0,5 m high, preferably less than 0,35 m high and/or weigh 30 kilograms or less, preferably weigh 25 kilo or less. In an embodiment, the beams and the blocks have the same height, preferably providing a flush upper surface.

In an embodiment, the dimensions of the beams and blocks are selected to enable assembling a prefab construction element wherein the beams are parallel and rows of the mycelium blocks extend between the beams and wherein the heart to heart distance between the beams is 600 millimeter (mm) plus or minus 20 mm, preferably plus or minus 2 mm, or 300 mm plus or minus 10 mm, preferably plus or minus 1 mm.

In an embodiment, the beams have a cross-section with inclined side walls, e.g. have a V-shaped or trapezoid cross-section, and the blocks have a complementary cross-section with inclined walls, e.g. the side walls of the beams and the side walls of the extend at the same or substantially the same inclination.

In an embodiment, the blocks, after placing, abut and preferably interlock, e.g. have complementary portions, e.g. stepped portions or tongue and groove portions, such that the complementary portion of a first block interlocks with the complementary portion of a neighboring block.

In an embodiment, the growth of the fungus in the blocks has been stopped, e.g. by means of heating, reduced pressure, freezing, radiation and/or drying, before assembling the prefab construction element.

In an embodiment, the beam defining the enclosure, the top plate and/or the base plate are made of wood, fiberboard, plywood, or other cellulose based material.

An embodiment, in particular intended as a roof panel, comprises vertical battens and optionally horizontal battens that are fixed to at least one of the base plate and the top plate.

The invention also relates to a construction element, such as a roofing, flooring or wall panel, assembled by the method or kit described above.

In an embodiment, the beams are parallel and rows of the mycelium composite blocks extends between the beams and the heart to heart distance between the beams is 600 millimeter (mm) plus or minus 20 mm, preferably plus or minus 2 mm, or 300 mm plus or minus 10 mm, preferably plus or minus 1 mm.

In an embodiment, the mycelium has a thickness of at least 15 centimeters, preferably at least 20 centimeters.

Many roofing, flooring, or wall panels have a thickness of at least 20 cm, often 25 centimeters or more, and are provided with rock wool or glass wool to provide insulation. By using a relatively thick layer of the mycelium, the panel exhibits sufficient heat and noise insulation, without requiring a further material, such as rock wool or glass wool.

Suitable substrates for creating mycelium composite include wood materials, e.g. particles, such as saw dust and wood shavings, and materials from grain, maize, rice, or hemp.

Materials that can be added, e.g. up to a total amount of 40 wt% of the substrate in total, to the substrate include vegetable materials such as cucumber, peppers, grass, reed, beer broth, potato steam peel, root pulp and used growing substrates from greenhouses. Other examples are polystyrene, plastics, and cardboard materials, as well as inorganic materials such as perlite and vermiculite, preferably to obtain a substrate with a low carbon footprint to replace materials with a high carbon footprint.

Another embodiment comprises the inclusion of reinforcements, such as reinforcing fibers, e.g. filaments or staple fibers, or rods or beams in the mixture and/or the inclusion of chunks of mycelium composite in the mixture.

The at least one fungus is preferably a white rot fungus and preferably one that grows relatively quickly and/or is able to accept materials that are strange to its habitat.

In an embodiment, the fungus or at least one of the fungi is selected from the group consisting of Pleurotus ostreatus, Pleurotus eryngii, Stropharia Rugosoannulata, Trametes versicolor, Ganoderma Lucidum, Phanerochaete chrysosporium, Bjerkandera adusta, Lentinula edodes, Pycnoporus cinnabarinus, Pycnoporus sanguineus, Grifola frondosa, Schizophyllum commune, Neolentinus lepideus, and Heterobasidiom annosum.

Suitable nutrients include sugar, oatflakes, flour, rejected food, and human and animal hair.

The fungus digests the nutrient components in the substrate over a period of time sufficient to grow hyphae and to allow the hyphae to form a network of interconnected mycelia cells through and/or around the discrete particles and/or the chunks in the substrate thereby bonding the discrete particles and/or chunks together to form a (further) mycelium composite and optionally bonding the composite to the walls of the enclosure.

The chunks can be prepared by shredding a mycelium composite. In an embodiment, the chunks are dried, e.g. by means of heat and/or vacuum e.g. in a drying chamber. The chunks of mycelium composite enable a construction and/or insulation material that, compared to the initial composite, comprises (interstitial) cavities and/or that has significantly improved insulation properties. Further, the chunks facilitate accelerated manufacture of further composites, in that the chunks can be dried at a first location and/or a first point in time and used as a raw material for a further mycelium composite at another location and/or a later point in time. Thus, the further mycelium composite already comprises for at least a substantial part dried mycelium composite and thus requires less or no drying.

In an embodiment, the mycelium composite has a thermal conductivity, lambda (λ), of 0,037 W/mK or less, preferably 0,032 W/mK or less and/or a specific weight in a range from 100 to 200 kg/m3, preferably in a range from 120 to 180 kg/m3, and/or a porosity in a range from 10% to 50%, preferably 12% to 40%, preferably 15% to 30%.

In another embodiment, the ratio of the weight of the panel and the weight of the mixture, i.e. the weight of the panel divided by the weight of the mixture is smaller than 0,6, preferably smaller than 0,5, preferably smaller than 0,45 and/or the rafters have a width less than 25 mm, preferably less than 20 mm.

WO 2021/180948 relates to "a method of manufacturing a prefab construction element (11), preferably a load-bearing element, for frame construction, such as wood frame construction, comprising the steps of providing a roofing, flooring or wall panel (2), which panel (2) comprises an enclosure (3), providing a fungus and a substrate, introducing or preparing a mixture (10) of the fungus and the substrate, in the enclosure (3) and allowing the fungus to grow to form a network of hyphae through the mixture (10) and into the walls (4-7) of the enclosure (3) to form a mycelium composite, and drying the composite while it remains in the enclosure (3) of the panel (2)."

CN 108505636 relates to "a lightweight composite organic heat preservation and sound insulation prefabricated board for fabricated buildings. The lightweight composite organic heat preservation and sound insulation prefabricated board for the fabricated buildings comprises two organic sound absorption boards, a damping silicone rubber layer, two fiber cement pressure plates, wherein the two organic sound absorption boards are made of wood organic matter, white rot fungus hyphae and foaming agents; the damping silicone rubber layer is arranged between the two organic sound absorption boards, and is used for being connected with the organic sound absorption boards and cutting off the transmission of a sound wave; and the two pieces of fiber cement pressure plates are arranged on the outside side of the organic sound absorption boards."

ES 2 497 415 relates to a "Procedure for the growth of organic and biodegradable structures from agricultural waste and mushroom mycelium, and its use as insulating components in construction, characterized by its design and manufacture of coherent thermal insulation structures with certain rigidity, 100% organic and biodegradable, using for this agricultural waste (straw, wood shavings, leaves, seed husks ...) and seeds of different species of fungus (pleurotus ostreatus, lentinula edodes, ganoderma lucidum ...)."

The invention will now be explained in more detail with reference to the figures, which schematically show embodiments according to the present invention.
Figure 1 is a perspective view of a composite beam according to the present invention.
Figure 2 is a perspective view illustrating a method step according to the present invention, to wit placing mycelium blocks between parallel beams.
Figure 3 is a cross-section of a prefab construction element according to the present invention.
Figure 4A and 4B are perspective views of a floor and a roof panel according to the present invention.
Figure 5 is perspective view of part of a building comprising a floor panel according to the present invention.
Figure 6A is perspective view of a building comprising floor, roof, and wall panels according to the present invention.
Figure 6B shows an enlarged detail of a window in one of the wall panels of the building in Figure 6A.
Figures 7A and 7B are perspective views of a window frame and a detail of that frame.
Figure 8 schematically illustrates the mounting of a window frame in a wall panel according to the present invention.

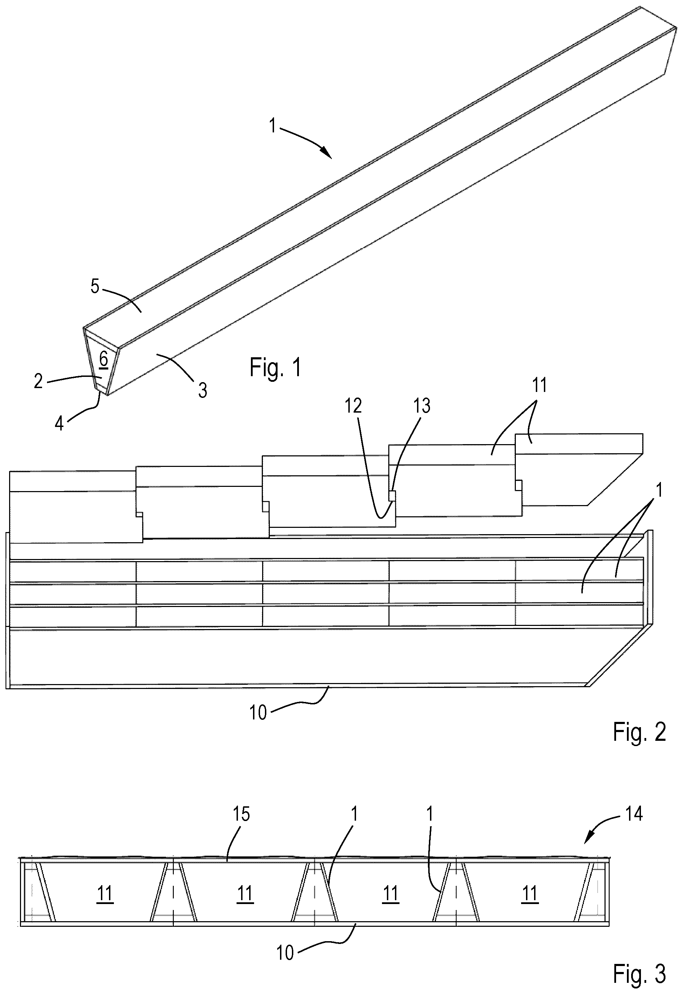

Figure 1 shows a load-bearing beam 1 made from four wooden elements, such a wooden planks 2-5 or boards having a thickness in a range from 20 to 55 mm, enclosing a mycelium composite 6. The beam 1 was made by forming an elongated enclosure having e.g. a V-shaped cross-section of three of the planks 2-4. Subsequently, a substrate, such as a blend of hemp, foliage, and sawdust was mixed with a fungus (inoculum), for instance Pleurotus ostreatus, optionally at least one nutrient, such as oatflakes, and water and the mixture was introduced into elongated enclosure. Next, the enclosure was covered, e.g. with an impermeable foil or tarpaulin, and the temperature of the mixture was maintained in a range from 15 to 24°C, for example 20°C. The fungus was allowed to grow, e.g. for a period in a range from 50 to 120 hours, preferably in a range from 70 to 110 hours, for example 100 hours, to form a network of hyphae through the mixture and into the walls of the enclosure to form a mycelium composite 6.

When the mycelium composite was considered at or near optimum, in terms of strength, stiffness and durability in a dried state, the fungus was killed by heating and drying the prefab construction element and the mycelium composite in it. The elongated enclosure was closed by means of a fourth plank 5, thus completing the load-bearing beam.

Figure 2 shows five load-bearing beams 1 placed in parallel and attached to a base plate 10. Rows of mycelium blocks 11 are placed between the beams and the blocks, after placing, interlock, e.g. have complementary portions, such as, in this example, stepped portions 12, 13. Further, in the finished construction element 14, shown in Figure 3, the cross-section of the blocks in the lateral direction is complementary to the cross-section of the beams. Thus, in the longitudinal direction of the construction element, i.e. the direction parallel to the beams, the blocks seals against each other and, in the lateral direction, the blocks seal against the beams, further improving the insulation properties of the construction element. A cover, e.g. a top plate 15, is placed over the beams and blocks and fastened to the beams, e.g. by means of an adhesive or mechanical fasteners, such as nails.

Further, in this example, as shown in Figure 3, the heart to heart distance between the beams is 600 mm and the width and height of the element are 2500 mm and 288 mm, respectively. The length of the element is 5 meters. In this example, the beams at the sides of the construction element have straight sides, to enable neighbouring construction elements to abut.

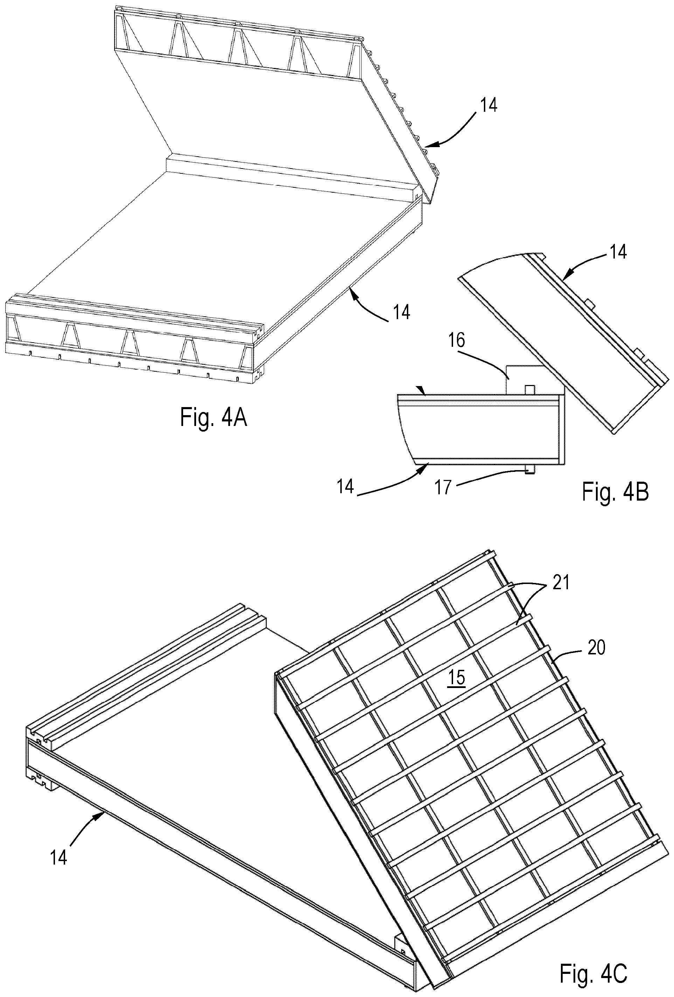

Figures 4A to 4C show a flooring panel 14 and a roof panel 14 inclined relative to the flooring panel. The roofing panel is mounted to the flooring panel by means of a grooved strip 16, which in turn has been fastened to the flooring panel by means of dowels 17, that were installed at the building site. The roofing panel is provided with vertical battens 20 fixed to the top plate 15 of the roofing panel and with horizontal battens 21 fixed to the vertical battens.

The present invention enables or at least facilitates large scale use of mycelium in prefab load-bearing and/or insulating construction elements, in that, on the one hand, the blocks can be produced in a relatively efficient, reproducible, homogenous and/or relatively fast manner and, on the other hand, the load-bearing prefab elements can be made in large sizes and preferably of at least 50 volume-percent of mycelium, preferably at least 70 volume-percent of mycelium. Also, the present invention provides a modern and in principle 100% organic construction element and kit that in principle fit substantially seamlessly in current prefab logistics.

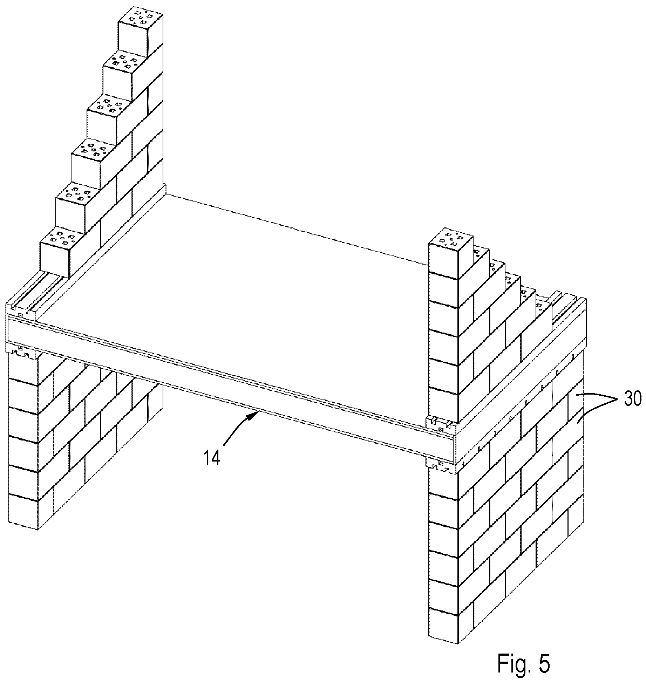

Figure 5 shows part of a building comprising a floor panel according to the present invention and building blocks 30 as disclosed in European patent application 23194713.6, thus entirely made from wood and mycelium.

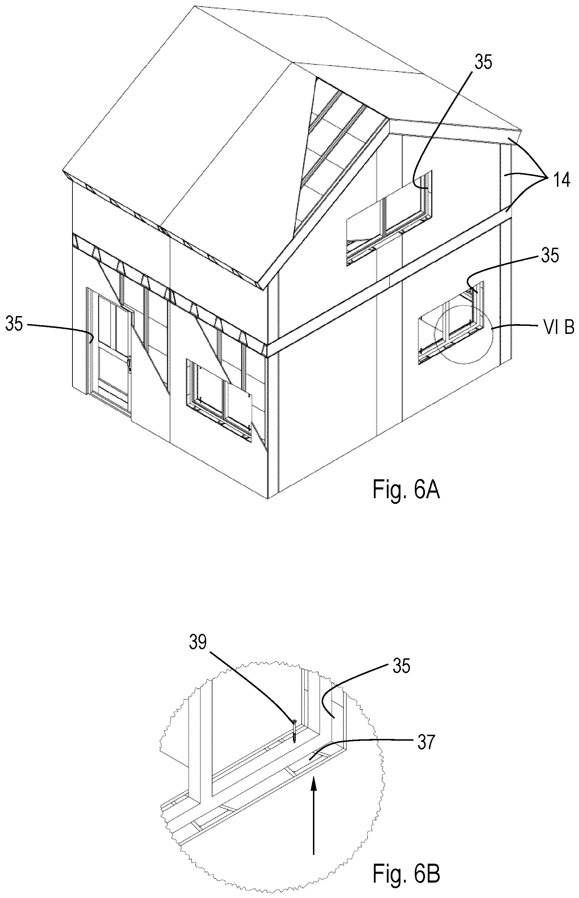

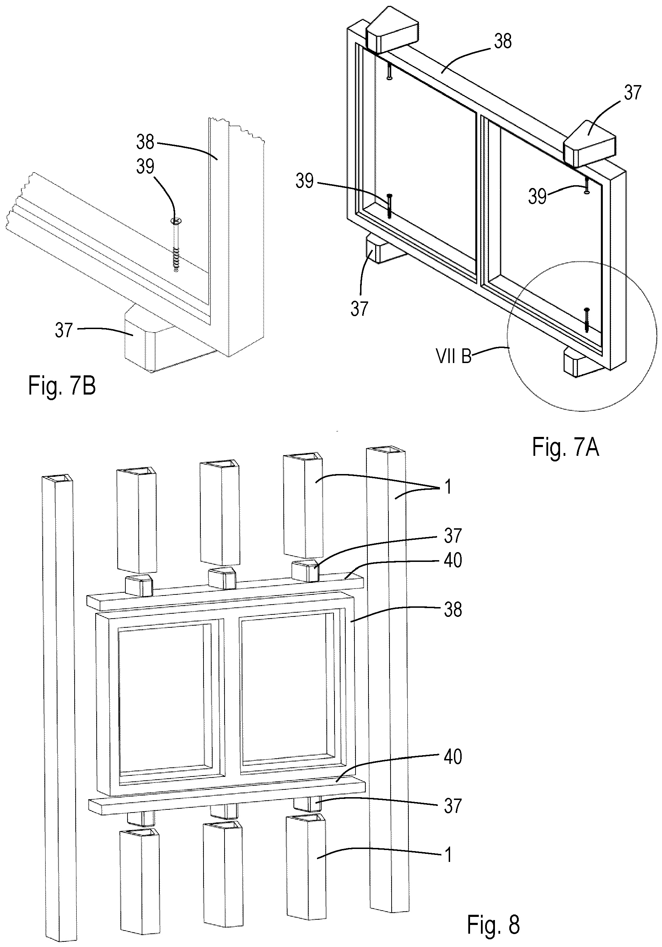

Figure 6A shows a building, specifically a house, built from floor, roof, and wall panels 14 according to the present invention. A number of the wall panels 14 comprise an opening 35 or part of an opening for a window or door. Mounting elements, e.g. solid blocks 37 made from wood, wood fibers, or a synthetic material, have been placed on the mycelium blocks between the beams and/or the mycelium in the ends of the beams. To this end, the blocks have a cross-section that corresponds to the cross-section of the blocks of mycelium composite or, preferably and in the examples shown in Figures 6A to 8, to the cross-section of the elongated enclosure of the beams 1. By removing mycelium composite from the mycelium block(s) and/or from the elongated enclosure(s) - or by providing less mycelium in the first place - over a height that corresponds to the height of the mounting elements, the latter, once placed in the resulting cavities, sit flush with the edge of the ends of the beams 1 and thus flush with the opening 35 for a window or door. The mounting elements are fixed in place e.g. by means of nails driven through an outer wall of the panel or beams or by means of an adhesive. Once the mounting elements have been placed and fixed to the beams, a window frame 38 is placed in the opening and fixed, e.g. by screws 39, to the mounting elements 37. This is also illustrated in Figures 7A and 7B, which show four mounting elements placed in and fixed in the ends of the beams, without showing the panel and beams themselves. A screw 39 is applied through the window frame 38 and into each of the mounting elements 37.

Figure 8 illustrates the use of a lintel 40, where a plurality of mounting elements 37 is fixed to the lintel, e.g. by means of screws or nails, at a heart to heart distance equal to that of the beams. Top and bottom lintels are placed on and in the beams 1, with the mounting elements extending into the beams, facing upwards and downwards, respectively. The lintels are levelled and fixed in place. Subsequently, a window frame is placed in de opening and fixed, e.g. by screws, to the lintels.

The invention is not restricted to the above-described embodiments, which can be varied in a number of ways within the scope of the claims. For instance, the opening described above can also be applied in an indoors panel, e.g. a wall of floor panel inside the building, e.g. to provide a door, opening, or arch between rooms or a well for stairs.

## Claims

1. Method of manufacturing a prefab construction element, preferably a load-bearing element, such as a roofing, flooring or wall panel (14), comprising the steps of
providing one or more beams, preferably load-bearing beams (1),
providing a plurality of blocks (11) of mycelium composite, and
assembling the prefab construction element (14) from the one or more load-bearing beams (1) and a plurality of the blocks (11).

2. Method according to claim 1, comprising the steps of
placing two or more beams (1) alongside each other and
placing a plurality of the blocks (11) between these beams (11).

3. Method according to claim 1 or 2, comprising the step of interconnecting the beams (1).

4. Method according to any one of the preceding claims, comprising the step of placing a cover (15) over the placed beams (1) and blocks (1) and fastening the cover (15) to the beams (1) and/or to the blocks (11).

5. Method according to any one of the preceding claims, comprising the steps of providing an opening (35) in the construction element (14) and placing one or more mounting elements (37) between the beams (1) and/or in the ends of the beams (1).

6. Method according to claim 5, wherein the mounting elements (37) have a cross-section that corresponds to the cross-section of the blocks (11) of mycelium composite between the beams (1) or to the cross-section of the elongated enclosures of the beams and preferably fit snugly between the beams (1) or in the elongated enclosures of the beams (1) and/or, after placing, sit flush with the edge of the ends of the beams (1).

7. Kit for manufacturing a prefab construction element, preferably a load-bearing element, such as a roofing, flooring or wall panel (14), comprising one or more beams, preferably load-bearing beams (1), a plurality of blocks (11)of mycelium composite, and preferably a base plate (10) or frame for interconnecting the beams (1) and/or mounting elements to be fitted between the beams and/or in the ends of the beams.

8. Method or kit according to any one of the preceding claims, wherein one or more of the load-bearing beams (1) comprises an elongated enclosure which enclosure contains a mixture of at least one fungus and a substrate, wherein a network of hyphae has formed through the mixture and into the walls of the enclosure to form a mycelium composite (6).

9. Method or kit according to any one of the preceding claims, wherein one or more beams (1) are at least 2 meter (m) long, preferably at least 4 m long, and/or less than 0,4 m wide, preferably less than 0,3 m wide, and/or less than 0,5 m high, preferably less than 0,35 m high.

10. Method or kit according to any one of the preceding claims, wherein the blocks (11) are at least 0,3 m long, preferably at least 0,4 m long, and/or at least 0,3 m wide, preferably less than 0,4 m wide, and/or less than 0,5 m high, preferably less than 0,35 m high and/or weigh less than 30 kilo, preferably weight 25 kilo or less.

11. Method or kit according to any one of the preceding claims, wherein the beams (1) have a cross-section with inclined side walls (2, 3) and the blocks (11) have a complementary cross-section with inclined walls.

12. Method or kit according to any one of the preceding claims, wherein the blocks (11), after placing, interlock.

13. Method or kit according to any one of the preceding claims, wherein the growth of the fungus in the blocks has been stopped and/or wherein vertical battens (20) and optionally horizontal battens (21) are fixed to at least one of the base plate (10) and the top plate (15) and/or wherein the beam (1) defining the enclosure, the top plate (15) and/or the base plate (10) are made of wood, fiberboard, plywood, or other cellulose based material.

14. Construction element, such as a roofing, flooring or wall panel (14), assembled by the method or kit according to any one of the preceding claims, optionally comprising an opening, such as a window, door, or well, and a frame, such as a window or door frame, positioned in the opening.

15. Construction element (14) according to claim 14, wherein the beams (1) are parallel and rows of the mycelium blocks (11) extends between the beams and wherein the heart to heart distance between the beams is 600 millimeter (mm) plus or minus 10 mm, preferably plus or minus 2 mm, or 300 mm plus or minus 5 mm, preferably plus or minus 1 mm.
